# EUROPEAN PATENT APPLICATION

(11) **EP 2 366 358 A1**
(43) Date of publication of application: **21.09.2011**
(21) Application number: 10156637.0
(22) Date of filing: 16.03.2010
(51) Int. Cl.: A61C 5/00, A61C 13/00

(54) **Dental appliance**

(71) Applicant: Remedent, 9831 Deurle (BE)
(72) Inventor: Jacquemyns, Evelyne, 9830, Sint-Martens-Latem (BE)
(74) Representative: Quintelier, Claude

(57) **Abstract**

Dental appliance comprising two or more dental prostheses 4 being pre-shaped to fit at least a part of a subject's dental arch 1 and being suitable for forming, when applied to said dental arch 1, a predetermined dental arch, said appliance further comprising positioning means (7, 9) provided to assist a dentist in simultaneously positioning said two or more dental prostheses 4 on said dental arch 1, wherein said positioning means (7, 9) are provided to allow a re-positioning from one with respect to an adjacent one of said two or more dental prostheses 4 after the latter have been simultaneously positioned on said dental arch 1.

## Description

The present invention relates to a dental appliance comprising two or more dental prostheses being pre-shaped to fit at least a part of a subject's dental arch and being suitable for forming, when applied to said dental arch, a predetermined dental arch, said appliance further comprising positioning means provided to assist a dentist in simultaneously positioning said two or more dental prostheses on said dental arch. The present invention further relates to a method for manufacturing a dental appliance comprising two or more dental prostheses and a positioning means provided to assist a dentist in simultaneously positioning said two or more dental prostheses on a subject's dental arch, said method comprising the steps of:
- acquiring three-dimensional data relating to said subject's dental arch;
- manufacturing, using said three-dimensional data, two or more dental prostheses that are provided to fit said dental arch and that are determined so as to form, when applied to said dental arch, at least a part of a predetermined dental arch;
- assembling said positioning means and said two or more dental prostheses thereby forming an assembly that fits said dental arch.

It is known that the surfaces of teeth sometimes become permanently stained, decayed or damaged. Several techniques have been developed to repair or improve the appearance and function of such teeth.

In a method referred to as cosmetic bonding, a thin dental prosthesis such as a veneer or a facet having a shape and curvature matching i.a. the outline, shape and surface curvature of a tooth to be refaced, is bonded to the front surface of the tooth. Using a conventional technique, each of the dental prostheses is individually crafted and individually applied to the front surface of a tooth. The materials are specially selected to match the colour and translucency of natural teeth. Dental prostheses such as veneers improve the cosmetic appearance of stained and/or damaged teeth, and are typically bound to the surface of the natural teeth using an adhesive.

The conventional practice during bonding of a dental prosthesis to a tooth is for a dentist to hold the dental prosthesis in place during bonding with his or her finger. In particular the positioning and fitting of the dental prosthesis normally entails the dentist holding onto the dental prosthesis with fingers of one hand, and then manipulating the dental prosthesis in the subject's mouth to position, fit and bind the dental prosthesis. Often, the positioning, fitting and bonding requires fingers of the other hand to be placed into the subject's mouth as well. This practice is cumbersome. For example, a typical bonding process requires the use of a light probe to cure a light sensitive adhesive. However, the finger of the dentist obscures the dental prosthesis, and makes it difficult to both see the dental prosthesis and direct the light to the portion of the dental prosthesis that the dentist wants to cure.

Moreover, the dentist may need to reposition the dental prosthesis prior to curing the adhesive using a single finger in a wet protective glove that may have sticky adhesive on one part and may be slippery on another part can have the tactile sensation impaired, slipping in relation to the dental prosthesis. Slipping can cause an error in the positioning of the dental prosthesis, thereby compromising the integrity and aesthetics of the dental prosthesis. Adhesives that are typically used to apply prostheses to teeth are adhesives that cure almost instantaneously after exposure to an appropriate light beam. This results in that if margins that are not properly sealed, the dental prosthesis must be removed by drilling away both the dental prosthesis and adhesive from the surface of the tooth, causing subject discomfort, a prolonged procedure, and replacement by a new dental prosthesis.

Furthermore, since dental prostheses are often produced extern, the dentist receives the dental prostheses already (pre)formed in a package. If from the package it is not unambiguously clear which dental prosthesis corresponds to which tooth, or if due to falling of the package or dental prostheses the mutual relationship, defining which dental prosthesis corresponds to which tooth, is disturbed, the dentist has to find out by himself which dental prosthesis corresponds to which tooth. In particular for less experienced dentist, finding this out proves to be very hard and many mistakes are made. These mistakes lead to dental prostheses that have to be removed, causing subject discomfort, a prolonged procedure, and often replacement by a new dental prosthesis.

In the patent application US2007/0292821, which is considered the closest prior art, it is proposed to place the dental prostheses in a tray thereby forming an assembly that fits the teeth of the subject's dental arch. According to this prior art, the different dental prostheses are manufactured in one piece so as to be interconnected. Using the tray, the interconnected dental prostheses can be positioned onto the teeth of a subject all at the same time. To this end, the dental prostheses and the tray are preferably produced based on a 3D model of the subject's dental arch so as to fit this dental arch. This results in a rather short application of dental prostheses to the subject's teeth, which is an advantage because a longer application to the teeth causes more discomfort to the subject. Because the dental prostheses are interconnected, they can not be interchanged and thus there will be no issue regarding which prosthesis corresponds to which tooth.

Applying the interconnected dental prostheses using the tray raises problems for the dentist in that there is nearly no possibility to interfere in the positioning, fitting and binding stage of the dental prostheses. Namely once the adhesive is applied to the interconnected dental prostheses, and the latter have been simultaneously positioned onto the teeth using the tray, the dentist can't but wait until the adhesive has cured so that the tray can be removed. After removal of the tray, there is no possibility to adjust the position of the prostheses because the adhesive has already cured and because the dental prostheses are interconnected. Because of these reasons, the dentist depends on the quality of the, in most cases, externally produced dental prostheses and tray for what concerns positioning and fitting precision of the dental prostheses with respect to the teeth.

It is an object of the invention to provide dental appliance that allows the dentist to come to a more aesthetic application of the dental prostheses, and a method for manufacturing such dental appliance.

To this end, the dental appliance according to the invention is characterised in that said positioning means are provided to allow a re-positioning from one with respect to an adjacent one of said two or more dental prostheses after the latter have been simultaneously positioned on said dental arch. Because a dentist, using a dental appliance according to the present invention, can re-position a dental prosthesis using the positioning means, the dentist can improve the application process of the prostheses. After having simultaneously positioned the prostheses onto the teeth, the dentist can adjust the position of the prostheses to his own discretion. This results in first instance in a fast application of the prostheses to the teeth while in second instance maintaining the flexibility to individually adjust the position of each of the prostheses.

According to a first embodiment of the invention, said positioning means comprise a connecting member provided to interconnect said two or more dental prostheses in such a manner as to allow a re-positioning from one with respect to an adjacent one of said two or more dental prostheses. In such dental appliance, a connecting member interconnects the dental prostheses. Thereby, the mutual relationship between the dental prostheses is determined and thus clear identification as to which prosthesis corresponds to which of the teeth, is possible. Because the prostheses are interconnected, they can more easily be applied simultaneously to the teeth of the subject. The connecting member furthermore allows a re-positioning from one with respect to an adjacent one of the prostheses. This results in that the dentist, using the dental appliance according to the invention, can correct the position of each of the prostheses with respect to one another.

Preferably, said connecting member is pre-shaped so as to match with said predetermined dental arch. The pre-shaped connecting member results in that the dental prostheses connected thereto are positioned in a predetermined manner that fits the dental arch of the subject. This considerably simplifies the simultaneous application of the prostheses to the teeth.

Preferably, the connecting member is a flexible elongated element provided for being connected to each of said two or more dental prostheses. Due to the flexibility of the elongated element, prostheses can be interconnected while still maintaining a certain ability of re-positioning one with respect to another. Because the element is elongated, it can be positioned extending over all the dental prostheses thereby providing the possibility to interconnect all the prostheses using only one element. The flexible elongated element is preferably connected to each prosthesis in a mid portion thereof. A dental prosthesis, in particular a veneer, comprises a front surface, on which surface a mid portion can be defined. Connecting the flexible elongated element only to a mid portion of the prostheses results in that the elongated element, between two adjacent connecting points on the prostheses, extends freely over these prostheses, which free portion in combination with the flexibility allows the adjacent prostheses to move one with respect to another.

Preferably, said connecting member is formed of a transparent material. The effect is that the dentist can see through the connecting member. This makes it easier for the dentist to see what he or she is doing thus this connecting member being transparent contributes to the ease of positioning and re-positioning of the prostheses.

According to a second embodiment of the invention, said positioning means comprise a substantially rigid tray that matches with said predetermined dental arch and that is provided to receive each of said two or more dental prostheses, said tray being provided, after having received said two or more dental prostheses, to release the received prostheses upon exertion of a force which is smaller than an initial adhesive force between said dental prostheses and said dental arch. In such a dental appliance, the rigid tray can be regarded as a carrier in which the prostheses are placed in a substantially loose manner. The tray is provided to release the prostheses upon exertion of a force smaller than the initial adhesive force between the dental prostheses and the dental arch of the subject. Since the initial adhesive force is weak, being hardly strong enough to prevent the prostheses to fall off the dental arch, the prostheses are loosely received by the tray. However, preferably the prostheses are held by the tray strong enough to prevent the prostheses to fall out due to gravity force. The tray being provided to receive all the prostheses allows the dentist to simultaneously apply the prostheses onto the teeth of the subject. The prostheses are received by the tray and are kept within the tray due to a first adhesive force between the tray and the prostheses. After the prostheses are positioned onto the teeth, a second adhesive force can be distinguished between the prostheses and the teeth. According to the invention, the second adhesive force is larger than the first adhesive force. This results in that the tray is provided to directly release the prostheses after initial simultaneous application, thereby allowing a re-positioning from one with respect to an adjacent one of the prostheses. As a result, the dentist can, using the dental appliance according to the invention, correct the position of each of the prostheses with respect to one another after having simultaneously applied the prostheses onto the teeth.

According to a third embodiment of the invention, the dental appliance comprises both a connecting member as described above and a tray as described above, which tray is provided to receive said connecting member interconnecting said dental prostheses. Simultaneously positioning the prostheses onto the teeth is easier with the tray than with the connecting member, while re-positioning the prostheses with respect to one another is easier with the connecting member than with the tray. Combining the tray and the connecting member in one dental appliance thereby combines the advantages of both systems, being the ease of simultaneous application provided by the tray and the ease of re-positioning after application provided by the connecting member. The combination furthermore proves to provide a synergetic effect in that the disadvantages of each of the first and second embodiment are eliminated in the combination.

The invention further provides a method for manufacturing such dental appliance. To this end, the method according to the present invention is characterised in that said assembling is performed in such a manner as to obtain an assembly allowing a re-positioning from one with respect to an adjacent one of said two or more dental prostheses. Because a dentist, using a dental appliance manufactured according to the present invention, is able following the method, to re-position a dental prosthesis, the dentist can contribute to the quality of the application process of the prostheses. After having simultaneously positioned the prostheses onto the teeth, the dentist can adjust the position of the prostheses to his own discretion. This results in first instance in a fast application of the prostheses to the teeth while in second instance maintaining the flexibility to individually adjust the position of each of the prostheses. Thereby, the dental appliance being manufactured according to the present invention provides a combination of the advantages of the simultaneous application method according to the closest prior art, and the advantages of the conventional, individual application method, as described above.

Preferably, said step of acquiring said positioning means comprises manufacturing, using said three-dimensional data and using data relating to a shape of said manufactured two or more dental prostheses, a connecting member that matches with said predetermined dental arch, and wherein said step of assembling comprises connecting said connecting member to each of said two or more dental prostheses. Because the connecting member is manufactured based on the three-dimensional data and the shape of the manufactured prostheses, the connecting member fits the predetermined dental arch. This results in that when the assembly is made, this assembly fits the dental arch of the subject, thus making it easier for the dentist to simultaneously position the assembly to the teeth of the subject.

Preferably, said step of acquiring said positioning means comprises manufacturing, using said three-dimensional data and using data relating to a shape of said manufactured two or more dental prostheses, a substantially rigid tray that matches with said predetermined dental arch, that is provided to receive said two or more dental prostheses, and that is provided, after having received said two or more dental prostheses, to release the received prostheses upon exertion of a force which is smaller than an initial adhesive force between said dental prostheses and said dental arch, and wherein said step of assembling comprises placing said two or more dental prostheses in said tray. Manufacturing the dental appliance in such a manner, results in that the dentist is able to simultaneously position the prostheses onto the teeth of the subject thereby maintaining the possibility to re-position the prostheses after application.

The invention will now be described in more details with respect to the drawings illustrating some preferred embodiments of a dental appliance according to the present invention. In the drawings:
figure 1 shows an exploded view of a dental arch and a dental appliance according to the invention;
figure 2 shows a dental appliance according to the third embodiment of the invention;
figure 3 shows a dental appliance according to the first embodiment of the invention; and
figure 4 shows a dental appliance according to the second embodiment of the invention.

In the drawings a same reference number has been allocated to a same or analogous element.

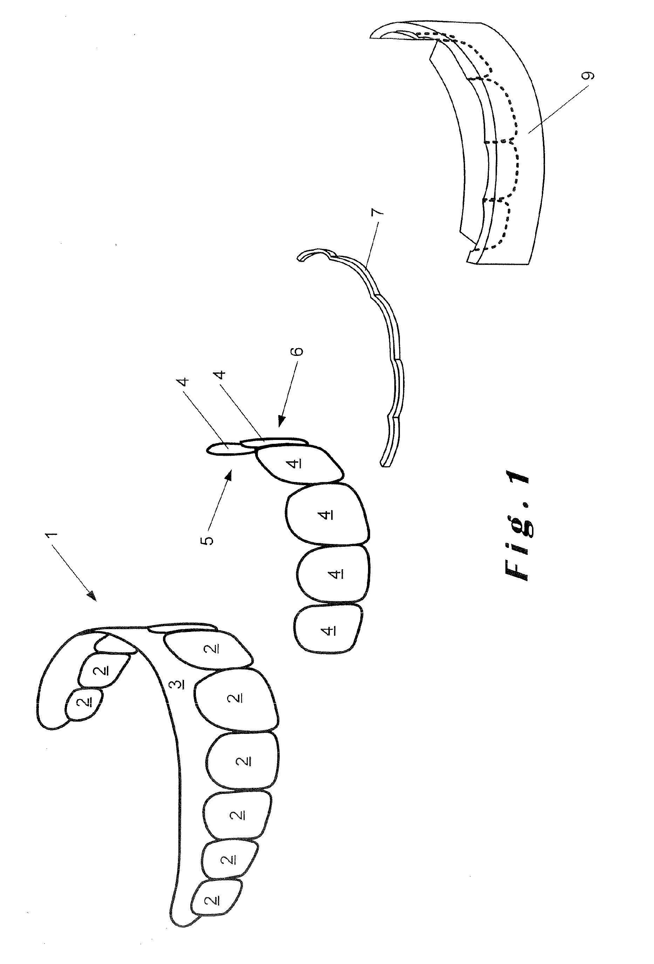

Figure 1 shows a part of a dental arch 1 of a person. The shown part of the dental arch 1 represents only the upper and front part of a complete dental arch of a person. For the purpose of understanding the invention, it will be sufficient to explain the dental appliance only with respect to this upper and front part of the dental arch, hereafter simply called dental arch 1. However it will be clear that the figures do not limit the invention, and those parts of the complete dental arch of a person that are not shown in the figures are also treatable using an appliance according to the present invention.

The dental arch 1 comprises a number of teeth 2 and comprises gum 3. The shape and dimensions of each of the teeth 2 is defined by the visible edges of the teeth on one side and the gum 3 on the other side. Sometimes the surface of teeth 2 becomes damaged, which negatively affects the visual appearance of the teeth 2. Such damages can affect the colour, shape or even the physical location of the teeth. Dental prosthesis, in particular veneers, are frequently used to improve the cosmetic appearance of such damaged teeth. The wording veneer is to be interpreted in this text as including laminate, facing, facet and the like. Furthermore, in this text the wording predetermined dental arch refers to the dental arch with the set of veneers applied thereto. The predetermined dental arch thereby implies that the veneers are positioned with respect to each other in a digitally modelled final application position.

Figure 1 shows a set of veneers 4. Each veneer 4 is preferably individually designed to cover a corresponding tooth in the dental arch 1. To this end, each veneer preferably shows dimensions that are substantially equal to the dimensions of the tooth which they should overlay. The veneer is preferably coloured to provide the cosmetic appearance that the person wishes to obtain or is coloured to correspond to the remaining teeth. Several techniques are known to produce veneers. The present invention is not limited to a particular technique. However there is a preferred method of producing such veneers in which method the veneers are digitally modelled and designed based on a 3D scan of at least a part of the dental arch of the person. After digitally modelling and designing the veneers, the latter are produced via cad/cam or via rapid prototyping or via other ways which allow producing physical objects out of a digital model.

The set of veneers are provided to be permanently applied to the dental arch by gluing, cementing or any other suitable technique available to the skilled person. To this end, each veneer comprises a connecting surface 5 and, opposite of the connecting surface 5, a front surface 6. The front surface 6 will provide the veneer with the cosmetic appearance as explained above. The connecting surface 5 is preferably shaped so that it is substantially equal in shape and curvature as the surface of the corresponding tooth 2. This results in a maximal connecting surface upon which the glue, cement or other suitable medium can be applied for connecting the veneer 4 with the tooth 2. Preferably the used connecting medium is glue that cures when exposed to a light source having a predetermined light frequency. Such glues are known and frequently used in dental applications because of the ease in use.

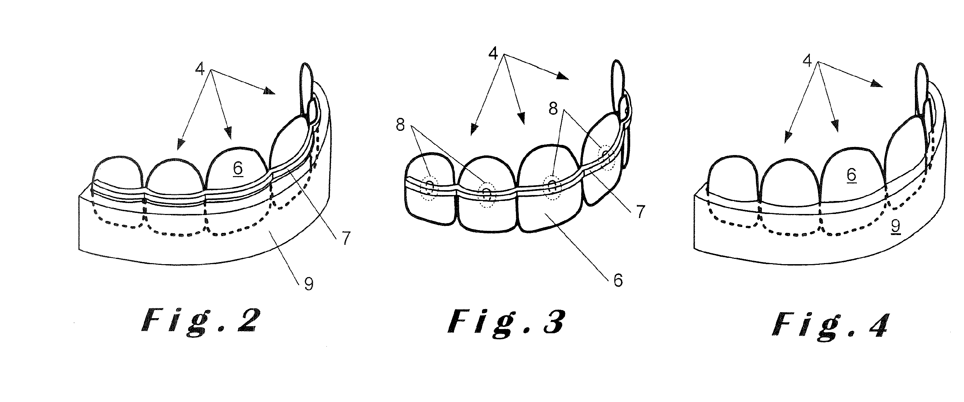

According to a first embodiment, as shown in figure 3, the dental appliance according to the invention comprises a connecting member 7 extending over a set of veneers 4. The connecting member 7 is formed as a flexible elongated member 7 that extends over, and can be connected to each of the veneers in the set of veneers 4. The connecting member 7 is connected to a veneer 4 in a mid portion 8 of the front surface 6 of the veneer 4. Preferably, the connecting member is glued to the veneer 4 using glue that can be completely removed from the veneer without substantial effort and without the need of damaging the veneer. The mid portion 8 of the veneer 4 is located at a distance of at least the lateral peripheral sides of the veneer 4. The flexible elongated member 7 that extends over a veneer and is only connected to the veneer in a mid portion thereof, thus freely extends over the lateral sides, adjacent to the mid portion, of the veneer. This freely extending of flexible elongated member between two connecting points on adjacent veneers result in that the veneers can be re-positioned one with respect to an adjacent one, namely the elongated member is not substantially hindered to move in between the two connecting points. Preferably, the total of front surface area 6 of a veneer 4, with respect to the mid portion 8 thereof, is larger then three times the mid portion 8, more preferably larger then five times the mid portion 8, most preferably larger then 10 times the mid portion 8.

Preferably, the flexible elongated member 7 shows in cross-section, at least one flat side, more preferably the elongated member is square in cross-section. The flat side, or one of the sides of the square, is provided to lay against the front surface 6 of the veneers. Preferably, the elongated member 7 is pre-shaped to match the predetermined dental arch. Thereby, the flexible elongated member 7 is provided to lay closely against the front surfaces 6 of the veneers 4 when they are in the final position, thus closely against the predetermined dental arch. This results in that when the veneers 4 are connected to the elongated member 7, without an external force, the veneers 4 are positioned in the predetermined final position, thus in the form of the predetermined dental arch.

The veneers 4 and flexible elongated member 7 are preferably manufactured based upon three-dimensional data from the dental arch 1 of the subject. After the acquiring of the three-dimensional data from the subject's dental arch 1, the data is processed so as to design two or more dental prostheses 4, in particular veneers 4. The veneers 4 are designed to form, when placed on the dental arch 1, at least a part of a predetermined dental arch thereby preferably improving the visual appearance of the dental arch of the subject. The designed dental prostheses 4 are manufactured using known techniques.

Several techniques are known to obtain three-dimensional data from a dental arch of a person, for example a three-dimensional photograph or a CAD/CAM scanning of a moulded model of the dental arch. Furthermore, techniques are known to design dental prostheses, in particular veneers. The present invention is not restricted to a particular technique of obtaining three-dimensional data, nor is it restricted to a particular technique of designing prostheses.

The predetermined dental arch is known from the three-dimensional data acquired from the dental arch 1 of the subject in combination with the data relating to the shape of the designed dental prostheses 4. The flexible elongated member 7 is preferably determined so that it matches the predetermined dental arch. The determined flexible elongated member 7 is manufactured after being digitally determined. Several ways are known to manufacture such a member that is digitally determined, for example via a CAD/CAM system or 3D-printing or rapid prototyping. In a final step, the manufactured flexible elongated member 7 is assembled with the manufactured dental prostheses 4 so as to form a dental appliance according to the invention. The elongated member 7 is assembled with the prostheses 4 by gluing the member to each of the prostheses in the mid portion 8 thereof.

In a second embodiment of the invention, as shown in figure 4, the dental appliance according to the invention comprises a substantially rigid tray 9 and a set of at least two dental prostheses 4, in particular veneers 4. The veneers 4 are positioned in the tray 9 so that they can be simultaneously fitted to the dental arch 1 of the subject. To this end, the tray is formed to receive the dental prostheses.

In general, when gluing a veneer to a tooth, at first, glue is to be applied to one or both of the veneer 4 and the tooth 2. Secondly, the veneer 4 is moved towards the tooth 2 so that the glue contacts both the veneer 4 and the tooth 2 thereby forming a film of glue between the latter. Thirdly, the veneer 4 is positioned correctly with respect to the tooth 2. After the correct positioning step, the glue can be cured for example by exposing the glue to a particular light source.

In the second of the above-mentioned steps of gluing a veneer 4 to a tooth 2, the glue contacts both the veneer 4 and the tooth 2 resulting in an initial adhesive force between veneer 4 and tooth 2. Preferably, this initial adhesive force prevents the veneer 4 from falling off the tooth 2 or more preferably prevents the veneer 4 from moving with respect to the tooth 2 without a substantial external force being applied to the veneer 4.

The tray 9 according to the present invention is provided to receive the dental prostheses 4 in a sequence corresponding to the final position at the dental arch. Furthermore the tray 9 is designed to release the dental prostheses 4 upon exertion of a force which is smaller than the initial adhesive force. This results in that the veneers 4, being simultaneously moved towards the teeth 2 using the tray 9, stick to the teeth 2 when the tray is removed. Thereby, an individual repositioning of a veneer 4 with respect to a tooth 2 is possible after the veneers 4 have been simultaneously moved onto the teeth 2.

The tray 9 is preferably determined using the three-dimensional data from the dental arch 1 of the subject in combination with the data relating to the shape of the designed dental prostheses 4, as explained with respect to the first embodiment. The determined tray 9 is manufactured in a next step. Several ways are known to manufacture such a tray 9 that is digitally determined, for example via a CAD/CAM system or 3D-printing or rapid prototyping. In a final step, the manufactured tray 9 is assembled with the manufactured dental prostheses 4 so as to form a dental appliance according to the invention. The tray 9 is assembled with the prostheses 4 by placing the prostheses 4 in the corresponding locations in the tray 9.

In a third embodiment of the present invention, as shown in figure 2, and as shown in an exploded view in figure 1, the dental appliance comprises a set of at least two dental prostheses 4, a flexible elongated member 7 and a tray 9. Thereby, the third embodiment is a combination of the first and the second embodiment described above. In the third embodiment, a flexible elongated member 7 is connected to each of the veneers 4 in a mid portion 8 thereof, similar to the first embodiment. Furthermore, the veneers 4 are placed in a tray 9 similar to the second embodiment. However in the third embodiment, the flexible elongated member 7 is placed so that it does not hinder the veneers 4 in being placed into the tray 9. To this end, the flexible elongated member 7 is connected to the veneers 4 in an area thereof that is located outside the tray 9, the veneers 4 being considered positioned in the tray 9. Preferably, the tray 9 is provided to receive the veneers 4 thereby only contacting the lower half of the veneers 4, more preferably only one third of the veneers 4. This results in that when the flexible elongated member 7 is glued to the veneers 4 in a mid portion 8 thereof, where the mid portion 8 is preferably located slightly shorter to the top edge of the veneer 4 then to the lower edge of the veneer 4, the elongated member 7 does not hinder the veneers 4 in being positioned in the tray 9.

In producing the third embodiment, preferably both the flexible elongated member 7 and the tray 9 are determined based upon three-dimensional data. Digitally determining both the tray and the elongated member, and simulating the assembly in the computer ensures that no collision occurs between flexible elongated member 7 and tray 9 when assembling the latter with the veneers 4.

In use, the first embodiment has the advantage that the veneers are connected to each other by the connecting member. This ensures that, even when the dental appliance falls onto the ground, the location of each veneer with respect to the others is fixed. Furthermore, the connecting member allows a re-positioning from one to an adjacent one of the veneers. Thereby, a dentist can correct a position of a veneer with respect to a tooth when applying the veneers to the dental arch of a patient. However in this first embodiment, it requires some skill to simultaneously apply the dental appliance onto the teeth of the subject because of the flexibility of the connecting member.

In use, the second embodiment has the advantage that it is easy to simultaneously apply the veneers onto the teeth of the subject. Furthermore, because of the tray being rigid and the veneers being located therein in a predetermined position, the veneers are applied to the teeth in this predetermined position. Thus a minimal re-positioning is required after such application. Re-positioning is possible because the initial adhesive force is larger then the force that keeps the veneers into the tray. Thereby, the tray releases the veneers easily when applied to the teeth, and the dentist can to its own discretion individually correct the position of each veneer. In this second embodiment, there is the risk that, when the dental appliance falls onto the ground, the tray releases the veneers. Then the position of the veneers with respect to each other is not fixed anymore, and the dentist has to find the correct position of each veneer. In practise, this is a considerable problem.

In use, the third embodiment combines the advantages of both the first and the second embodiment while eliminating the disadvantages of both embodiment. Therefore, the third embodiment is preferred over the first and the second embodiment.

Although with respect to the first and the third embodiment, the flexible elongated member is described as pre-shaped so as to match the predetermined dental arch, it should be noted that also a straight or non-pre-shaped flexible elongated member can be used as a connecting member according to the present invention. The invention is therefore not limited to pre-shaped elongated members.

## Claims

1. Dental appliance comprising two or more dental prostheses (4) being pre-shaped to fit at least a part of a subject's dental arch (1) and being suitable for forming, when applied to said dental arch, a predetermined dental arch, said appliance further comprising positioning means (7 and/or 9) provided to assist a dentist in simultaneously positioning said two or more dental prostheses (4) on said dental arch (1), **characterised in that** said positioning means (7 and/or 9) are provided to allow a re-positioning from one with respect to an adjacent one of said two or more dental prostheses (4) after the latter have been simultaneously positioned on said dental arch (1).

2. Dental appliance according to claim 1, wherein said positioning means comprise a connecting member (7) provided to interconnect said two or more dental prostheses (4) in such a manner as to allow a re-positioning from one with respect to an adjacent one of said two or more dental prostheses.

3. Dental appliance according to claim 2, wherein said connecting member (7) is pre-shaped so as to match with said predetermined dental arch.

4. Dental appliance according to claim 2 or 3, wherein said connecting member (7) is a flexible elongated element (7) provided for being connected to each of said two or more dental prostheses (4).

5. Dental appliance according to claim 4, wherein said flexible elongated element (7) is substantially square in cross-section.

6. Dental appliance according to any of the claims 4-5, wherein said flexible elongated element (7) is provided for being connected to each of said two or more dental prostheses (4) in a mid portion (8) thereof.

7. Dental appliance according to any of the claims 2-6, wherein said connecting member (7) is formed of a transparent material.

8. Dental appliance according any of the previous claims, wherein said positioning means comprise a substantially rigid tray (9) that matches with said predetermined dental arch and that is provided to receive each of said two or more dental prostheses (4), said tray (9) being provided, after having received said two or more dental prostheses (4), to release the received prostheses (4) upon exertion of a force which is smaller than an initial adhesive force between said dental prostheses (4) and said dental arch (1).

9. Dental appliance according to claim 8 and any of the claims 2-7, wherein said tray (9) is provided to receive said connecting member (7) interconnecting said dental prostheses.

10. Dental appliance according to any of the previous claims, wherein said dental prostheses (4) are veneers.

11. Method for manufacturing a dental appliance comprising two or more dental prostheses (4) and a positioning means (7 and/or 9) provided to assist a dentist in simultaneously positioning said two or more dental prostheses (4) on a subject's dental arch (1), said method comprising the steps of:
• acquiring three-dimensional data relating to said subject's dental arch (1);
• manufacturing, using said three-dimensional data, two or more dental prostheses (4) that are provided to fit said dental arch and that are determined so as to form, when applied to said dental arch, at least a part of a predetermined dental arch;
• assembling said positioning means (7 and/or 9) and said two or more dental prostheses (4) thereby forming an assembly that fits said dental arch;
**characterised in that** said assembling is performed in such a manner as to obtain an assembly allowing a re-positioning from one with respect to an adjacent one of said two or more dental prostheses (4).

12. Method for manufacturing a dental appliance according to claim 11, wherein before said step of assembling, the method comprises a step of manufacturing, using said three-dimensional data and using data relating to a shape of said manufactured two or more dental prostheses (4), a connecting member (7) that matches with said predetermined dental arch, and wherein said step of assembling comprises connecting said connecting member (7) to each of said two or more dental prostheses (4).

13. Method for manufacturing a dental appliance according to claim 12, wherein said step of connecting said connecting member (7) comprises a step of positioning said connecting member (7) with respect to said two or more dental prostheses (4) so that said connecting member (7) extends over a front surface (6) of each of said prostheses (4), and comprises a further step of gluing said positioned connecting member (7) to each of said prostheses (4) in a mid portion (8) thereof.

14. Method for manufacturing a dental appliance according to any of the claims 11-13, wherein before said step of assembling, the method comprises a step of manufacturing, using said three-dimensional data and using data relating to a shape of said manufactured two or more dental prostheses (4), a substantially rigid tray (9) that matches with said predetermined dental arch, that is provided to receive said two or more dental prostheses(4), and that is provided, after having received said two or more dental prostheses (4), to release the received prostheses (4) upon exertion of a force which is smaller than an initial adhesive force between said dental prostheses (1) and said dental arch (1), and wherein said step of assembling comprises placing said two or more dental prostheses (4) in said tray (9).
